# EUROPÄISCHE PATENTSCHRIFT

(11) **EP 1 480 963 B1**
(45) Veröffentlichungstag und Bekanntmachung des Hinweises auf die Patenterteilung: **02.05.2007**
(21) Anmeldenummer: 03702270.4
(22) Anmeldetag: 28.02.2003
(51) Int. Cl.: C07D 295/20, C07D 211/60

(54) **VERFAHREN ZUR HERSTELLUNG VON 3-AMIDINOPHENYLALANIN-DERIVATEN**
METHOD FOR PRODUCING 3-AMIDINOPHENYLALANINE DERIVATIVES
PROCEDES DE PRODUCTION DE DERIVES 3-AMIDINOPHENYLALANINE

(30) Priorität: 28.02.2002 CH 347022002
(43) Veröffentlichungstag der Anmeldung: 01.12.2004
(73) Patentinhaber: Wilex AG, 81675 München (DE)
(72) Erfinder: ZIEGLER, Hugo, CH-4108 Witterswil (CH); WIKSTROEM, Peter, CH-5073 Gipf-Oberfrick (CH)
(74) Vertreter: Weickmann, Franz Albert
(86) Internationale Anmeldenummer: PCT/CH2003/000147
(87) Internationale Veröffentlichungsnummer: WO 2003/072559

(56) Entgegenhaltungen:
- WO-A-00/17158
- CH-A- 689 611
- STURZEBECHER J ET AL: "3-Amidinophenylalanine-based Inhibitors of Urokinase" BIOORGANIC & MEDICINAL CHEMISTRY LETTERS, OXFORD, GB, Bd. 9, Nr. 21, 1. November 1999 (1999-11-01), Seiten 3147-3152, XP004181024 ISSN: 0960-894X
- JUDKINS B D ET AL: "A VERSATILE SYNTHESIS OF AMIDINES FROM NITRILES VIA AMIDOXIMES" SYNTHETIC COMMUNICATIONS, MARCEL DEKKER, INC., BASEL, CH, Bd. 26, Nr. 23, 1996, Seiten 4351-4367, XP002059866 ISSN: 0039-7911
- JENDARLLA H ET AL: "EFFICIENT KG-SCALE SYNTHESIS OF THROMBIN INHIBITOR CRC 220" TETRAHEDRON, ELSEVIER SCIENCE PUBLISHERS, AMSTERDAM, NL, Bd. 51, Nr. 44, 30. Oktober 1995 (1995-10-30), Seiten 12047-12068, XP000644579 ISSN: 0040-4020 in der Anmeldung erwähnt
- MONGE A ET AL: "NEW QUINOXALINE ND PYRIMIDO4,5-BQUINOXALINE DERIVATIVES. POTENTIAL ANTIHYPERTENSIVE AND BLOOD PLATELET ANTIAGGREGATING AGENTS" JOURNAL OF HETEROCYCLIC CHEMISTRY, HETEROCORPORATION. PROVO, US, November 1989 (1989-11), Seiten 1623-1626, XP001079354 ISSN: 0022-152X in der Anmeldung erwähnt

## Beschreibung

Die vorliegende Erfindung betrifft Verfahren zur Herstellung von 3-Amidinophenylalanin-Derivaten unter Verbesserung der chemischen Gesamtausbeute sowie Erhöhung der Enantiomerenreinheit. Diese 3-Amidinophenylalanin-Derivate stellen eine Klasse von hochwirksamen Inhibitoren der Urokinase dar (WO 00/1758).

Die in WO 00/17158 beschriebenen Synthesemethoden zur Herstellung von Urokinase-Inhibitoren beinhalten eine Methode zur Umwandlung der Cyanfunktion von substituierten 3-Cyanophenylalanin-Derivaten in eine Amidinfunktion. Nachteilig ist dabei die durch eine mehrstufige Umwandlung der Nitrilfunktion in die Amidinofunktion verursachte ungenügende Ausbeute und die Verwendung von karzinogenen Reagenzien, wie Schwefelwasserstoff und Methyliodid, und die Freisetzung von Methylmercaptan als hochtoxisches Gas als Nebenprodukt. Dieses Verfahren ist mit einem höheren apparativen Aufwand und zusätzlichen Trennverfahren verbunden. Zusätzlich muss mit Racemisierung und damit geringerem enantiomeren Überschuss gerechnet werden.

In Tetrahedron 51,12047-68 (1995)(Schema 3) ist die Umwandlung einer para-ständigen Nitrilgruppe in eine Amidinofunktion mit Hydroxlamin-Hydrochlorid/Triethylamin und anschliessender Pdkatalytischer Hydrierung (10 bar / AcOH / 50°C) beschrieben. Allerdings sind die Reaktionsbedingungen so harsch, dass die chemische Ausbeute sowie die chemische Reinheit nicht befriedigen. Über die Enantiomerenreinheit werden keine Angaben gemacht.

In Tetrahedron Letters 40, 7067-71 (1999) wird eine neue milde Methode zur Herstellung von aromatischen Amidinen aus Nitrilen beschrieben, welche gegenüber allen bis dannzumal bekannten Methoden Vorteile zeigen soll. Dabei wird die Reaktion mit Acetylcystein und Ammoniak bei Temperaturen von ca. 60°C vorgenommen. Der Nachteil dieser Methode liegt darin, dass vernünftige Ausbeuten nur mit π-Elektronen armen (= π-Elektronen ziehenden) Aromaten erzielt werden können.

In Bioorg. Med. Chem. Lett. 9, 3147-52 (1999) (Schema, Route B) wird die Umwandlung einer 3-Cyanfunktion von substituiertem Phenylalanin (vgl. Schema 1 (III) mit R¹=OH) in die 3-Amidinofunktion beschrieben. Dabei wird das Nitril zunächst durch Umsetzung mit Hydroxylaminhydrochlorid in das entsprechende Oxyamidin und in einem weiteren Schritt mit Acetanhydrid in das Acetoxyamidin überführt. Nach anschließender Reaktion der freien Säuregruppe zum Amid kann die aktivierte Verbindung in einem vierten Schritt mit Wasserstoff über Pd-Katalysatoren zum 3-Amidinophenylalanin-Derivat [vgl. Schema 1 (I)] umgesetzt werden.

Überraschenderweise wurde nun gefunden, dass die Umwandlung der aromatischen 3-Cyanfunktion von substituierten Phenylalanin Derivaten der allgemeinen Formel (III) in die 3-Amidinofunktion entsprechender Verbindungen der allgemeinen Formel (I) mit Hydroxylamin und anschliessende Reduktion des Oxyamidins der allgemeinen Formel (IIa) mit Wasserstoff über Pd-C (10%) oder Reduktion des mit Acetanhydrid in situ hergestellten entsprechenden Acetyloxyamidins mit Ammoniumformiat über Pd-C (10%) unter milden Reaktionsbedingungen bewerkstelligt werden kann (siehe das nachstehende Schema 1). Dabei werden die Reaktionsprodukte unter einem minimen apparativen Aufwand in hoher chemischen Ausbeute und Reinheit sowie ohne Racemisierung erhalten.

Weiter ist in EP 0 739 886 A2 ein Verfahren zur Herstellung von 4-Amidrazono-phenylalanin Derivaten aus den entsprechenden Nitrilen beschrieben. Dabei wird das Nitril zunächst in das entsprechende Thioamid umgewandelt, welches über ein Thioimidoester-Derivat so aktiviert wird, dass es mit Hydrazin zum Hydrazonoamid (Amidrazon) reagiert.

Eine direkte Überführung der Nitrilgruppe in ein Amidrazon ist in J. Heterocyclic Chem. 26, 1623 (1989) beschrieben. Dabei werden mit Cyano substituierte x-Elektronenmangel-Heteroaromaten (d.h. für einen nukleophilen Angriff begünstigte Cyanogruppen) mit Hydrazin während einiger Stunden gekocht, wobei das Amidrazon in mässigen Ausbeuten anfällt.

Überraschenderweise wurde nun weiterhin gefunden, dass sich auch ein nicht aktiviertes Nitril, das nicht an eine Elektronen ziehende Gruppe gebunden ist, unter ähnlichen Bedingungen mit Hydrazin umsetzen lässt. So können die Verbindungen der Formel (III) in guten Ausbeuten zu den Amidrazonen der Formel (IIb) umgesetzt werden (siehe Schema 1).

Überraschenderweise wurde auch gefunden, dass sich diese Amidrazone - analog zu den Amidoximen der Formel (IIa) - zu den Amidinen der Formel (I) reduzieren lassen (siehe Schema 1). Die direkte Überführung eines unsubstituierten Amidrazons zu einem unsubstituierten Amidin wurde bisher erst einmal beschrieben, nämlich bei Aza-Transfer Reaktionen zwischen Arylamidrazonen und Aryldiazoniumsalzen in Vestn. Slov. Kem. Drus. (1980), 27(3), 251-64.

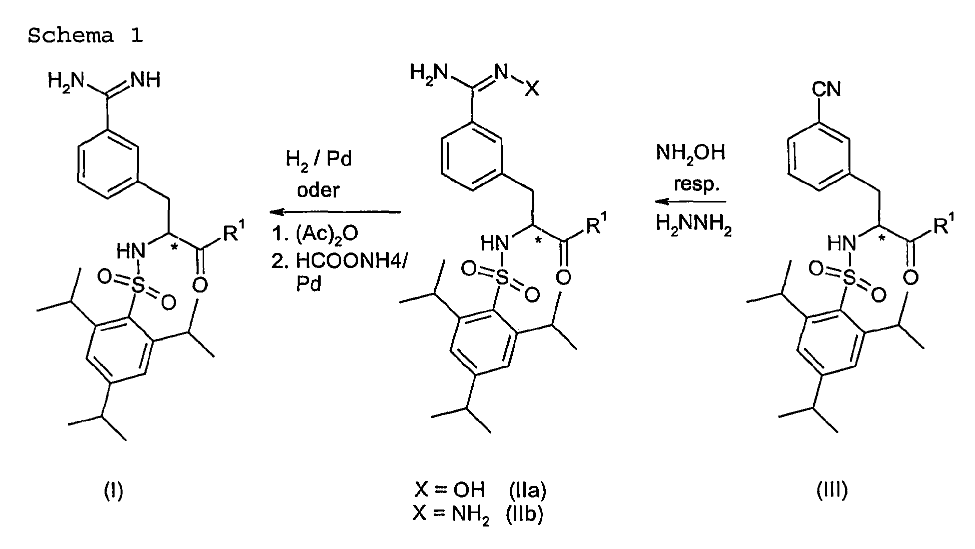

Gegenstand der Erfindung ist daher ein Verfahren zur Umwandlung von 3-Cyan-phenylalanin-Derivaten der allgemeinen Formel (III) in 3-Amidinoderivate der allgemeinen Formel I, oder ihre mit Säuren gebildeten Salze, die entweder als L-oder als D-konfigurierte Verbindungen vorliegen und in denen R¹
(a) eine Gruppe der Formel 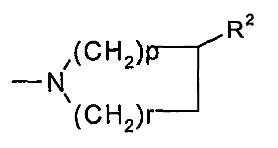 darstellt, in welcher entweder p = 1 und r = 2 und R² Benzyloxycarbonyl, Benzylaminocarbonyl oder 2-Thienylhydrazinocarbonyl sind oder p = 2 und r = 1 und R² Ethoxycarbonyl, 2-Propyloxycarbonyl, 2-Propylaminocarbonyl, Methylaminocarbonyl oder Methyl sind; oder
(b) eine Gruppe der Formel 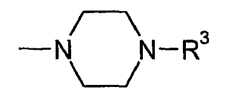 darstellt, in welcher R³ Methoxycarbonyl, Ethoxycarbonyl, Benzyloxycarbonyl, Dimethylaminocarbonyl, Acetyl oder Propionyl ist;
unter einem minimen apparativen Aufwand in hoher chemischen Ausbeute und Reinheit sowie ohne Racemisierung.

In einer ersten Ausführungsform des erfindungsgemässen Verfahrens erfolgt die Umwandlung der Nitrilgruppe in die Amidinofunktion über die Amidoxim-Zwischenstufe der allgemeinen Formel (IIa) mittels Hydroxylamin-Hydrochlorid in Gegenwart von Natriumcarbonat in alkoholisch-wässeriger Lösung bei Rückflusstemperatur, zweckmässigerweise durch 2- bis 20-stündiges, vorzugsweise 4- bis 10-stündiges Kochen einer Verbindung der Formel (III) mit einem bis zu 5-fachen Überschuss an Hydroxylamin-Hydrochlorid/0.5 - 0.6 Äquiv. Natriumcarbonat in alkoholisch-wässeriger, vorzugsweise ethanolisch-wässeriger Lösung. Die Umsetzung des Nitrils (III) mit dem Hydroxylamin-Hydrochlorid kann aber auch in Gegenwart von Triethylamin in alkoholischer Lösung, mit oder ohne Zusatz eines weiteren organischen Lösungsmittels, wie Methylenchlorid, bei Raumtemperatur erfolgen.

Die anschliessende Reduktion der Amidoximfunktion erfolgt entweder mit Wasserstoffgas oder mit Ammoniumformiat (welches zweckmässigerweise in einem mindestens 4-fachen Überschuss eingesetzt wird), entweder direkt ausgehend vom unsubstituierten Amidoxim, oder über das mit Acetanhydrid in Gegenwart von Salzsäure, zweckmässigerweise bei 20 bis 60°C, in situ hergestellte acetylierte Amidoxim, in alkoholisch/wässeriger Lösung, vorzugsweise ethanolisch/wässeriger Lösung, zweckmässigerweise im Verhältnis von 1:1 bis 20:1, vorzugsweise 3:1 bis 10:1, idealerweise 5:1, in Gegenwart von Pd/C, zweckmässigerweise 1 bis 50%, vorzugsweise 5 bis 30 % Pd/C (ca. 10%), zweckmässigerweise bei Normaldruck und einer Temperatur zwischen 10 und 50°C, vorzugsweise zwischen 20 und 30°C, idealerweise bei Raumtemperatur. Die Reduktion kann aber auch durch Hydrierung in Gegenwart von Pd/C in einer alkoholischen, mit Essigsäure oder Chlorwasserstoff versetzten Lösung unter einem Druck von etwa 1-3 bar erfolgen.

In einer zweiten Ausführungsform des erfindungsgemässen Verfahrens erfolgt die Umwandlung von 3-Cyan-phenylalanin Derivaten der allgemeinen Formel (III) in 3-Amidinoderivate der allgemeinen Formel I über die Amidrazon-Zwischenstufe der allgemeinen Formel (IIb) durch Kochen, zweckmässigerweise 2-bis 20-stündiges, vorzugsweise 4- bis 10-stündiges Kochen, einer Verbindung der Formel (III) mit überschüssigem Hydrazin in alkoholischer, vorzugsweise ethanolischer Lösung. Die Reduktion der Amidrazon-Zwischenstufe (IIb) zum entsprechenden Amidin (I) erfolgt unter gleichen Bedingungen wie diejenige ausgehend vom Amidoxim (IIa).

Ein weiterer Gegenstand der Erfindung sind die Verbindungen der in Schema 1 dargestellten allgemeinen Formel (II), insbesondere jene der nachstehenden Formeln (IV) und (V) 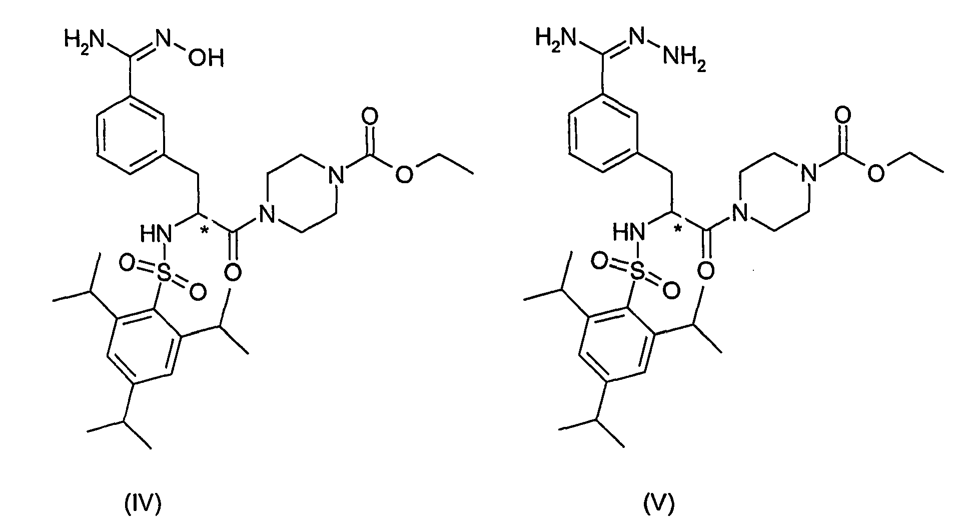 als (L)- oder (D)- Enantiomere, und als (E)- oder (Z)- Isomere oder (E/Z)-Gemische, und als freie Basen oder in Form ihrer mit Säuren gebildeten Salze.

In den nachfolgenden Ausführungsbeispielen werden die erfindungsgemäss verbesserten Syntheseverfahren und die Synthese neuer Zwischenprodukte näher erläutert, ohne aber die Erfindung einzuschränken.

Die Analyse der gemäss diesen Beispielen erhaltenen Eluate und Produkte wurde mit Proton-NMR, HPLC-Elektrospray-MS oder Elementaranalyse ausgeführt. Die Bestimmung der enantiomerischen Reinheit wurde mit an sich bekannten Verfahren mit der HPLC-Methode und chiralen analytischen Säulen bestimmt. Die Ausgangsverbindungen der allgemeinen Formel (III) und deren Herstellung sind bekannt (z.B. WO 00/17158)

### Beispiel 1:

### (A) N-α-2,4,6-Triisopropylphenylsulfonyl-3-oxamidino-(L)-phenylalanin-4-ethoxycarbonyl-piperazid

75.4 g (0.126 mol) N-α-2,4,6-Triisopropylphenylsulfonyl-3-cyan-(L)-phenylalanin-4-ethoxycarbonyl-piperazid wurden in 1.5 L Ethanol gelöst, die Lösung mit 32.5 g (0.47 mol) Hydroxylamin-Hydrochlorid und mit einer Lösung von 25.4 g (0.24 mol) Na₂CO₃ in 0.5 L Wasser versetzt und 6 Std. unter Rückfluss erhitzt (80°C). Das nach Abdestillieren des Lösungsmittels erhaltene Rohprodukt wurde in 1.5 L Ethylacetat aufgenommen und mit Wasser (3X 0.5 L) extrahiert, mit ges. NaCl gewaschen, über Na₂SO₄ getrocknet, filtriert und das Lösungsmittel abdestilliert. Ausbeute: 71.3 g (90%)

### (B) N-α-2,4,6-Triisopropylphenylsulfonyl-3-amidino-(L)-phenylalanin-4-ethoxycarbonyl-piperazid-Hydrochlorid

71.3 g (0.113 mol) des unter (A) erhaltenen N-α-2,4,6-Triisopropylphenylsulfonyl-3-oxamidino-(L)-phenylalanin-4-ethoxycarbonyl-piperazids wurden in 0.7 1 L Ethanol gelöst und die Lösung mit einer Suspension von 14.2 g 10% Palladiumkohle in 140 ml Wasser versetzt. Nach dem Einleiten von Wasserstoff bis zur Sättigung wurde bis zur vollständigen Umsetzung bei Normaldruck hydriert (ca. 5 Std). Die Suspension wurde über Celite filtriert, mit Ethanol/Wasser (9:1) gewaschen und das Lösungsmittel abdestilliert. Das erhaltene Rohprodukt wurde über Kieselgel 60 (Ethylacetat/2-Propanol, 8:2) gereinigt und anschliessend über Amberlite IRA-400 (Cl⁻-Form) in 2-Propanol/Wasser (8:2) in das entsprechende Hydrochlorid überführt. Ausbeute: 65.4g (89%) ee-Wert: 99.9% der (L)-Form.

### (C) N-α-2,4,6-Triisopropylphenylsulfonyl-3-amidino-(L)-phenylalanin-4-ethoxycarbonyl-piperazid-Hydrochlorid

71.3 g (0.113 mol) des unter (A) erhaltenen N-α-2,4,6-Triisopropylphenylsulfonyl-3-oxamidino-(L)-phenylalanin-4-ethoxycarbonyl-piperazids wurden in 0.71 L Ethanol gelöst, die Lösung mit 45.6 g (0.46 mol) Essigsäureanhydrid versetzt und 10 Min. bei RT gerührt. Dann wurden 0.46 L 1 N HCl zugegeben und die dabei warm werdende Lösung während 10 Min. nachgerührt. Nach dem Abkühlen auf Raumtemperatur wurden 29 g (0.46 mol) Ammoniumformiat zugesetzt und 5 Min. gerührt. Nach Zugabe einer Suspension von 14.2 g 10% Pd/C in 140 ml Wasser wurde während 24 Std. bei RT gerührt. Nach HPLC-Kontrolle der Reaktionsvollständigkeit wurde die Suspension über Celite abfiltriert, mit einem 1:9 Wasser/Ethanol Gemisch gewaschen und das Lösungsmittel abdestilliert. Das Rohprodukt wurde in 1.5 L EtOAc aufgenommen und mit 3 Portionen von je 0.5 L 1N HCl, Wasser und ges. NaCl gewaschen und über Na₂SO₄ getrocknet. Nach chromatographischer Reinigung über Kieselgel 60 mit (Ethylacetat/2-Propanol, 8:2) und anschliessender Ionenaustauschchromatographie über Amberlite IRA-400 (Cl⁻-Form) in 2-Propanol/Wasser (8:2) zur Ueberführung in das entsprechende Hydrochlorid wurde 62.5 g (85%) Produkt erhalten. ee-Wert: 99.9% der (L)-Form.

### Beispiel 2:

### (A) N-α-2,4,6-Triisopropylphenylsulfonyl-(L)-3-oxamidino-phenylalanyl-nipecotinsäure-benzylamid

2.3 g (3.6 mmol) Nα-2,4,6-Triisopropylphenylsulfonyl-(L)-3-cyan-phenylalanyl-nipecotinsäure-benzylamid wurden in 45 ml Ethanol gelöst, die Lösung mit 0.94 g (13.6 mmol) Hydroxylamin-Hydrochlorid und dann mit einer Lösung von 0.74 g (7 mmol) Na₂CO₃ in 15 ml Wasser versetzt und 6 Std. unter Rückfluss erhitzt (80°C). Das nach Abdestillieren des Lösungsmittels erhaltene Rohprodukt wurde in 100 ml Ethylacetat aufgenommen und mit Wasser (3X 30 ml) extrahiert, mit ges. NaCl gewaschen, über Na₂SO₄ getrocknet, filtriert und das Lösungsmittel abdestilliert. Ausbeute: 2.1 g (87%)

### (B) N-α-2,4,6-Triisopropylphenylsulfonyl-(L)-3-amidino-phenylalanyl-nipecotinsäure-benzylamid-hydrochlorid

2.1 g (3.1 mmol) des unter (A) erhaltenen N-α-2,4,6-Triisopropylphenylsulfonyl-(L)-3-oxamidino-phenylalanyl-nipecotinsäure-benzylamids wurden in 20 ml Ethanol gelöst und die Lösung mit einer Suspension von 0.4 g 10% Palladiumkohle in 5 ml Wasser versetzt. Nach Einleiten von Wasserstoff bis zur Sättigung wurde bis zur vollständigen Umsetzung bei Normaldruck hydriert (ca. 4 Std). Die Suspension wurde über Celite filtriert, mit Ethanol/Wasser (9:1) gewaschen und das Lösungsmittel abdestilliert. Das erhaltene Rohprodukt wurde über Kieselgel 60 (Ethylacetat/2-Propanol, 8:2) gereinigt und anschliessend über Amberlite IRA-400 (Cl⁻-Form) in 2-Propanol/Wasser (8:2) in das entsprechende Hydrochlorid überführt. Ausbeute: 1.74 g (85%), ee-Wert: 99.7% der (L)-Form

### Beispiel 3:

### (A) N-α-2,4,6-Triisopropylphenylsulfonyl-3-amidrazono-(L)-phenylalanin-4-ethoxycarbonyl-piperazid

75.4 g (0.126 mol) N-α-2,4,6-Triisopropylphenylsulfonyl-3-cyan-(L)-phenylalanin-4-ethoxycarbonyl-piperazid wurden in 1.5L Ethanol gelöst, die Lösung mit 18.1 g (0.47 mol) einer 100%-igen Hydrazinhydrat-Lösung versetzt und 6 Std. unter Rückfluss erhitzt (80°C). Das nach Abdestillieren des Lösungsmittels erhaltene Rohprodukt wurde in 1.5 L Ethylacetat aufgenommen und mit Wasser (3X 0.5 L) extrahiert, mit ges. NaCl gewaschen über Na₂SO₄ getrocknet, filtriert und das Lösungsmittel abdestilliert. Ausbeute: 65.7 g (83%).

### B) N-α-2,4,6-Triisopropylphenylsulfonyl-3-amidino-(L)-phenylalanin-4-ethoxycarbonyl-piperazid-Hydrochlorid

65.5g (0.104 mol) des unter (A) erhaltenen N-α-2,4,6-Triisopropylphenylsulfonyl-3-amidrazono-(L)-phenylalanin-4-ethoxycarbonyl-piperazids wurden in 0.66 L Ethanol gelöst und die Lösung mit einer Suspension von 13.1g 10% Palladiumkohle in 130 ml Wasser versetzt. Nach Einleiten von Wasserstoffgas bis zur Sättigung wurde bis zur vollständigen Umsetzung bei Normaldruck hydriert (ca. 5 Std). Die Suspension wurde über Celite filtriert, mit Ethanol/Wasser (9:1) gewaschen und das Lösungsmittel abdestilliert. Das erhaltene Rohprodukt wurde über Kieselgel 60 (Ethylacetat/2-Propanol, 8:2) gereinigt und anschliessend über Amberlite IRA-400 (Cl⁻-Form) in 2-Propanol/Wasser (8:2) in das entsprechende Hydrochlorid überführt. Ausbeute: 54.1 g (80%), ee-Wert: 99.8% der (L)-Form.

## Patentansprüche

1. Verfahren zur Herstellung von 3-Hydroxyamidino-phenylalanin Derivaten der allgemeinen Formel (IIa) 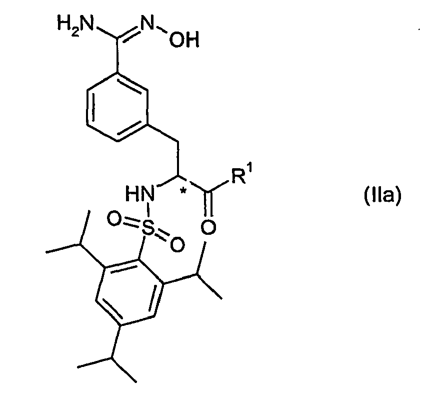 die als L- bzw. D-Enantiomere, und als (E)- oder (Z)- Isomere oder (E/Z)-Gemische, und als freie Basen oder in Form ihrer mit Säuren gebildeten Salze vorliegen und in denen R¹
(a) eine Gruppe der Formel 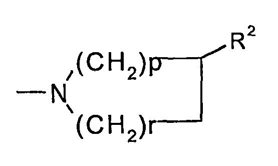 darstellt, in welcher entweder p = 1 und r = 2 und R² Benzyloxycarbonyl, Benzylaminocarbonyl oder 2-Thienylhydrazinocarbonyl sind oder p = 2 und r = 1 und R² Ethoxycarbonyl, 2-Propyloxycarbonyl, 2-Propylaminocarbonyl, Methylaminocarbonyl oder Methyl sind; oder
(b) eine Gruppe der Formel 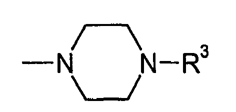 darstellt, in welcher R³ Methoxycarbonyl, Ethoxycarbonyl, Benzyloxycarbonyl, Dimethylaminocarbonyl, Acetyl oder Propionyl ist,
**dadurch gekennzeichnet, dass** man ein Nitril der allgemeinen Formel (III) 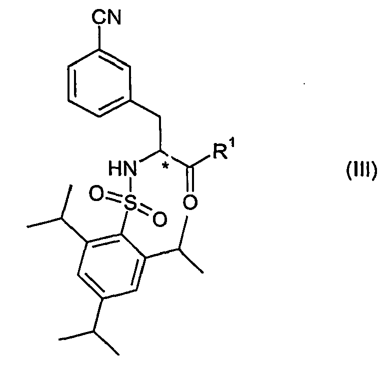 worin R¹ die obige Bedeutung hat,
mit Hydroxylamin-Hydrochlorid in Gegenwart von Natriumcarbonat in alkoholisch-wässeriger Lösung bei Rückflusstemperatur umsetzt.

2. Verfahren gemäss Anspruch 1, worin aber die Umsetzung des Nitrils der allgemeinen Formel III mit dem Hydroxylamin-Hydrochlorid in Gegenwart von Triethylamin in alkoholischer Lösung, gegebenenfalls unter Zusatz eines weiteren organischen Lösungsmittels, bei Raumtemperatur erfolgt.

3. Verfahren gemäss Anspruch 2, **dadurch gekennzeichnet, dass** als weiteres organisches Lösungsmittel Methylenchlorid verwendet wird.

4. Verfahren gemäss Anspruch 1, **dadurch gekennzeichnet, dass** ein bis zu 5-facher Überschuss an Hydroxylamin-Hydrochlorid eingesetzt wird und die Menge an Natriumcarbonat 0.5 bis 0.6 Moläquivalente bezogen auf das Hydroxylamin-Hydrochlorid beträgt.

5. Verfahren gemäss Anspruch 1 oder 4, **dadurch gekennzeichnet, dass** die Umsetzung in ethanolisch-wässeriger Lösung unter Rückfluss vorgenommen wird.

6. Verfahren zur Herstellung von 3-Amidrazono-phenylalanin Derivaten der allgemeinen Formel (IIb) 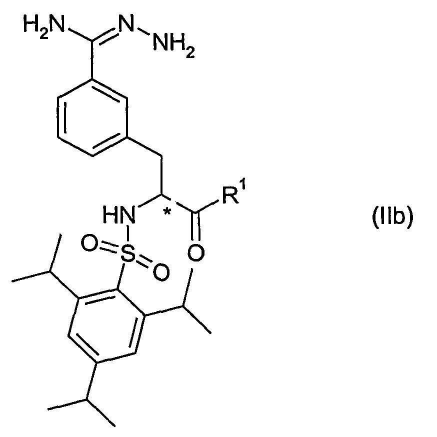 die als L- bzw. D-Enantiomere, und als (E)- oder (Z)-Isomere oder (E/Z)-Gemische, und als freie Basen oder in Form ihrer mit Säuren gebildeten Salze vorliegen und in denen R¹ die in Anspruch 1 angegebene Bedeutung hat, **dadurch gekennzeichnet, dass** man ein Nitril der in Anspruch 1 definierten allgemeinen Formel (III) mit überschüssigem Hydrazin in alkoholischer Lösung unter Rückfluss erhitzt.

7. Verfahren gemäss Anspruch 6 **dadurch gekennzeichnet, dass** in ethanolischer Lösung während 2 bis 20 Stunden, vorzugsweise während 4 bis 10 Stunden, unter Rückfluss erhitzt wird.

8. Verfahren zur Herstellung von 3-Amidino-phenylalanin Derivaten der allgemeinen Formel (I) 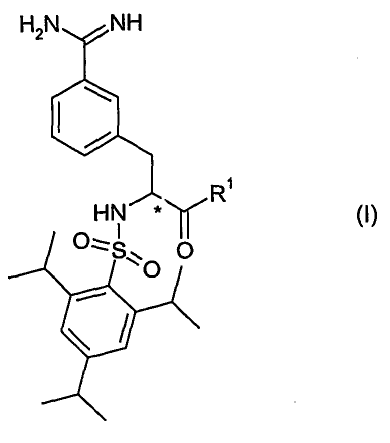 oder ihrer mit Säuren gebildeten Salze, die entweder als L-oder D-konfigurierte Isomere vorliegen und in denen R¹ die in Anspruch 1 angegebene Bedeutung hat, **dadurch gekennzeichnet, dass** man eine Verbindung der in Anspruch 1 definierten allgemeinen Formel (IIa) oder eine Verbindung der in Anspruch 6 definierten allgemeinen Formel (IIb), die als L- bzw. D-Enantiomere, und als (E)- oder (Z)-Isomere oder (E/Z)-Gemische vorliegen und in denen R¹ die in Anspruch 1 angegebene Bedeutung hat, in Gegenwart von Pd/C in einer alkoholisch-wässerigen Lösung mit Wasserstoff oder Ammoniumformiat reduziert.

9. Verfahren gemäss Anspruch 8, worin aber die Verbindung der in Anspruch 1 definierten allgemeinen Formel (IIa) oder der in Anspruch 6 definierten Formel (IIb) in Gegenwart von Pd/C in einer alkoholischen, mit Essigsäure oder Chlorwasserstoff versetzten Lösung unter einem Druck von 1-3 bar hydriert wird.

10. Variante des Verfahrens gemäss Anspruch 8, **dadurch gekennzeichnet, dass** man die Verbindungen der allgemeinen Formel (IIa) oder (IIb) mit Acetanhydrid in Gegenwart von Salzsäure in die entsprechenden Acetoxyamidino- resp. Acetaminoamidino-Derivate überführt und diese in Gegenwart von Pd/C in einer alkoholisch-wässerigen Lösung mit Wasserstoff oder Ammoniumformiat reduziert.

11. Verfahren gemäss Anspruch 10, worin aber die Acetoxyamidino- resp. Acetaminoamidino-Derivate in Gegenwart von Pd/C in einer alkoholischen, mit Essigsäure oder Chlorwasserstoff versetzten Lösung unter einem Druck von 1-3 bar hydriert werden.

12. Verfahren gemäss Anspruch 10, **dadurch gekennzeichnet, dass** die Überführung der Verbindungen der Formel (IIa) oder (IIb) in die entsprechenden Acetoxyamidino- resp. Acetaminoamidino-Derivate bei einer Temperatur von 20 bis 60°C erfolgt.

13. Verfahren gemäss einem der Ansprüche 8, 10 und 12, **dadurch gekennzeichnet, dass** in einer ethanolisch-wässerigen Lösung im Verhältnis von 1:1 bis 20:1, vorzugsweise im Verhältnis von 3:1 bis 10:1, insbesondere im Verhältnis von 5:1, reduziert wird.

14. Verfahren gemäss einem der Ansprüche 8, 10, 12 und 13, **dadurch gekennzeichnet, dass** in Gegenwart von 1 bis 50%, vorzugsweise in Gegenwart von 5 bis 30%, Pd/C (ca. 10%) reduziert wird.

15. Verfahren gemäss einem der Ansprüche 8, 10 und 12 bis 14, **dadurch gekennzeichnet, dass** bei einer Temperatur von 10 bis 50°C, vorzugsweise von 20 bis 30°C,reduziert wird.

16. Verfahren gemäss einem der Ansprüche 8, 10 und 12 bis 15, **dadurch gekennzeichnet, dass** mit einem mindestens vierfachen Überschuss an Ammoniumformiat reduziert wird.

17. Verbindungen der allgemeinen Formel (IIa) 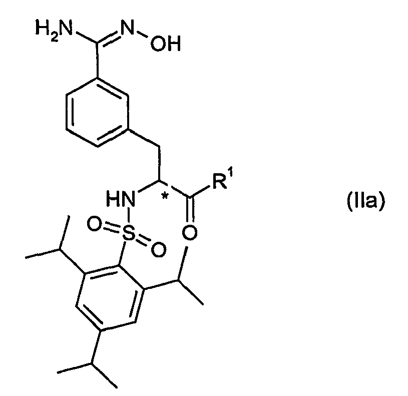 als (L)- oder (D)- Enantiomere, und als (E)- oder (Z)- Isomere oder (E/Z)-Gemische, und als freie Basen oder in Form ihrer mit Säuren gebildeten Salze, worin R¹ die in Anspruch 1 erwähnte Bedeutung hat.

18. Verbindungen der allgemeinen Formel (IIb) 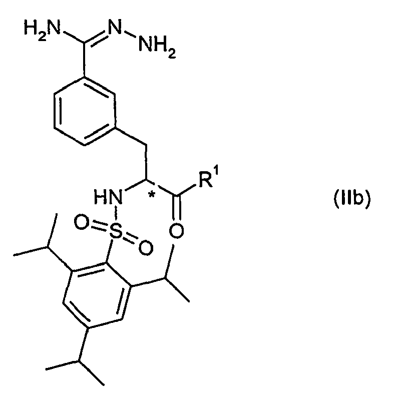 als (L)- oder (D)- Enantiomere, und als (E)- oder (Z)- Isomere oder (E/Z)-Gemische, und als freie Basen oder in Form ihrer mit Säuren gebildeten Salze, worin R¹ die in Anspruch 1 erwähnte Bedeutung hat.

19. Verbindungen gemäss Anspruch 17 bzw. Anspruch 18 der Formeln (IV) und (V) 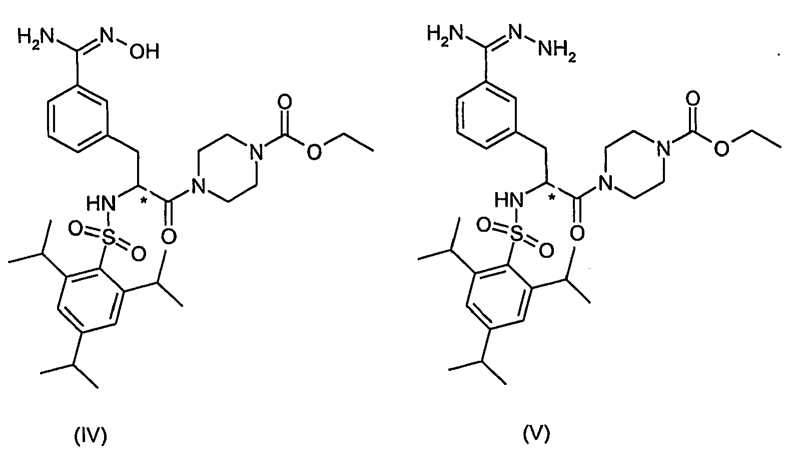 als (L)- oder (D) -Enantiomere, und als (E)- oder (Z)- Isomere oder (E/Z)-Gemische, und als freie Basen oder in Form ihrer mit Säuren gebildeten Salze.

## Claims

1. Process for the preparation of 3-hydroxyamidino-phenylalanine derivatives of general formula (IIa) 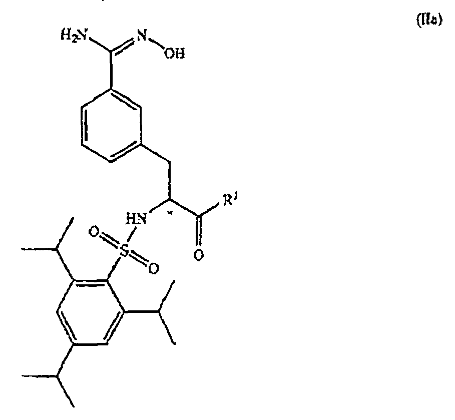 which are present as L- or D-enantiomers, (E)- or (Z)-isomers, or (E/Z)-mixtures and as free bases or the salts thereof formed with acids and wherein R¹ is
(a) a group of formula 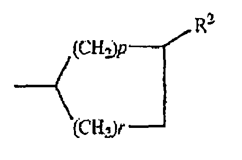 wherein p = 1 and r = 2 and R² is benzyloxycarbonyl, benyzlaminocarbonyl or 2-thienylhydrazinocarbonyl or p = 2 and r = 1 and R² is ethoxycarbonyl, 2-propyloxycarbonyl, 2-propylaminocarbonyl, methylaminocarbonyl or methyl; or
(b) a group of formula 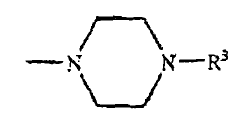
wherein R³ is methoxycarbonyl, ethoxycarbonyl, benzyloxycarbonyl, dimethylaminocarbonyl, acetyl or propionyl,
**characterised in that** a nitrile of general formula (III) 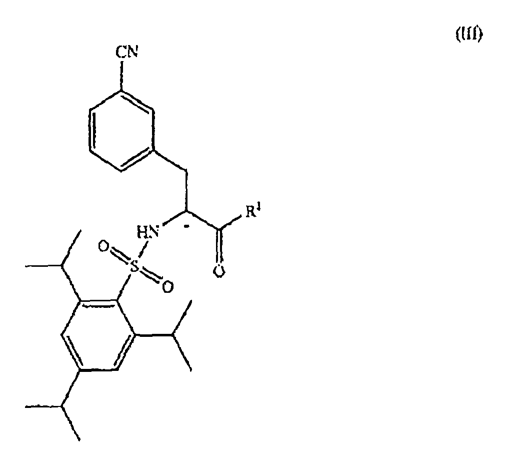 wherein R¹ is defined as above,
is reacted with hydroxylamine hydrochloride in the presence of sodium carbonate in alcoholic-aqueous solution at reflux temperature.

2. Process according to claim 1, wherein the reaction of the nitrile of general formula III takes place at room temperature with hydroxylamine hydrochloride in the presence of triethylamine in alcoholic solution, optionally with the addition of a further organic solvent.

3. Process according to claim 2, **characterised in that** the further organic solvent used is methylene chloride.

4. Process according to claim 1, **characterised in that** a one to five-fold excess of hydroxylamine hydrochloride is used and the quantity of sodium carbonate amounts to 0.5 to 0.6 mol equivalents relative to the hydroxylamine hydrochloride.

5. Process according to either claim 1 or claim 4, **characterised in that** the reaction is carried out in ethanolic-aqueous solution under reflux.

6. Process for the preparation of 3-amidrazono-phenylalanine derivatives of general formula (IIb) 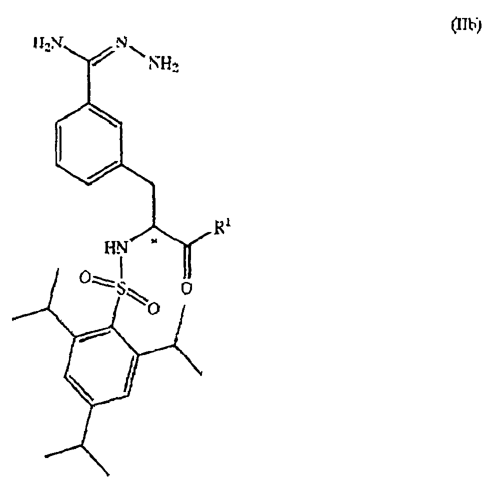 which are present as L- or D-enantiomers, (E)- or (Z)-isomers or (E/Z)-mixtures and as free bases, or as the salts thereof formed with acids and wherein R¹ is defined as in claim 1, **characterised in that** a nitrile of the general formula (III) defined in claim 1 is heated under reflux with an excess of hydrazine in alcoholic solution.

7. Process according to claim 6, **characterised in that** the refluxing in ethanolic solution lasts for 2 to 20 hours, preferably 4 to 10 hours.

8. Process for the preparation of 3-amidino-phenylalanine derivatives of general formula (I) 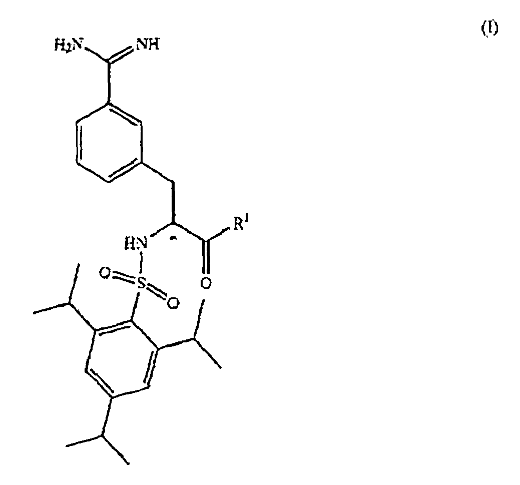 or the salts thereof formed with acids, which are present as either L- or D-configured isomers and wherein R¹ is defined as in claim 1, **characterised in that** a compound of the general formula (IIa) defined in claim 1 or a compound of the general formula (IIb) defined in claim 6, present as L- or D-enantiomers, as (E)- or (Z)-isomers or (E/Z)-mixtures, and wherein R¹ is defined as in claim 1, is reduced in the presence of Pd/C in an alcoholic-aqueous solution with hydrogen or ammonium formiate.

9. Process according to claim 8, wherein the compound of general formula (IIa) defined in claim 1 or of formula (IIb) defined in claim 6 is hydrogenated in the presence of Pd/C in an alcoholic solution mixed with acetic acid or hydrogen chloride under a pressure of 1 to 3 bar.

10. Variation of the process according to claim 8, **characterised in that** the compounds of general formula (IIa) or (IIb) are converted into the corresponding acetoxyamidino or acetaminoamidino derivatives respectively with acetanhydride in the presence of hydrochloric acid and these derivatives are reduced with hydrogen or ammonium formiate in the presence of Pd/C in an alcoholic-aqueous solution.

11. Process according to claim 10, wherein the acetoxyamidino or respectively acetaminoamidino derivatives are hydrogenated in the presence of Pd/C in an alcoholic solution mixed with acetic acid or hydrogen chloride under a pressure of 1 to 3 bar.

12. Process according to claim 10, **characterised in that** the conversion of the compounds of formula (IIa) or (IIb) into the corresponding acetoxyamidino or respectively acetaminoamidino derivatives takes place at a temperature between 20 and 60 °C.

13. Process according to any one of claim 8, 10 and 12, **characterised in that** the reduction takes place in an ethanolic-aqueous solution in a ratio of 1:1 to 20:1, preferably in a ratio of 3:1 to 10:1, particularly in a ratio of 5:1.

14. Process according to any one of claims 8, 10, 12 and 13, **characterised in that** the reduction takes place in the presence of 1 to 50 %, preferably in the presence of 5 to 30 % Pd/C (approximately 10 %).

15. Process according to any one of claims 8, 10 and 12 to 14, **characterised in that** the reduction takes place at a temperature of 10 to 50 °C, preferably 20 to 30 °C.

16. Process according to any one of claims 8, 10 and 12 to 15, **characterised in that** the reduction takes place with an at least four-fold excess of ammonium formiate.

17. Compounds of general formula (IIa) 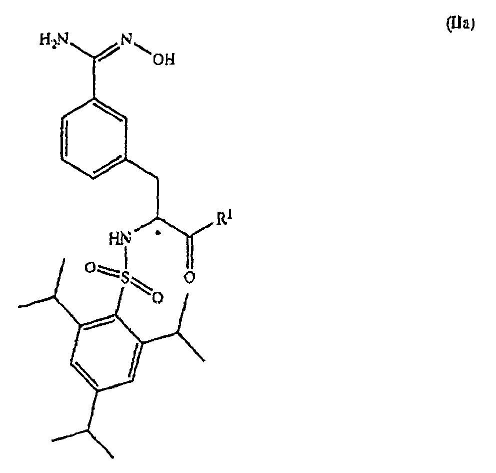 as (L)- or (D)-enantiomers, and as (E)- or (Z)-isomers or (E/Z)-mixtures, and as free bases or as the salts thereof formed with acids, wherein R¹ is defined as stated in claim 1.

18. Compounds of general formula (IIb) 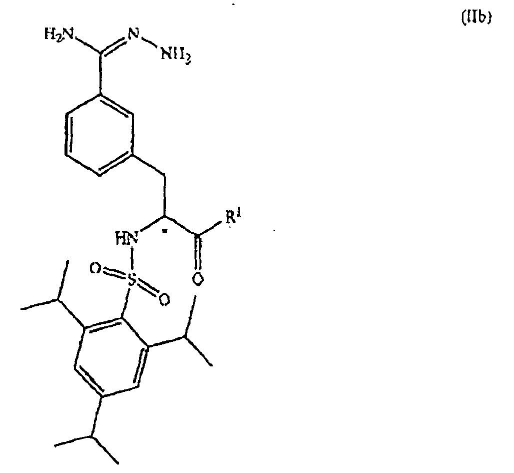 as (L)- or (D)-enantiomers, and as (E)- or (Z)-isomers or (E/Z)-mixtures, and as free bases or as the salts thereof formed with acids, wherein R¹ is defined as stated in claim 1.

19. Compounds according to claim 17 or respectively claim 18, corresponding to formula (IV) and formula (V) respectively 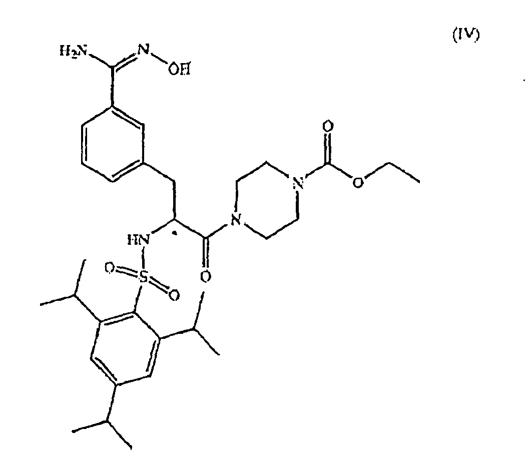 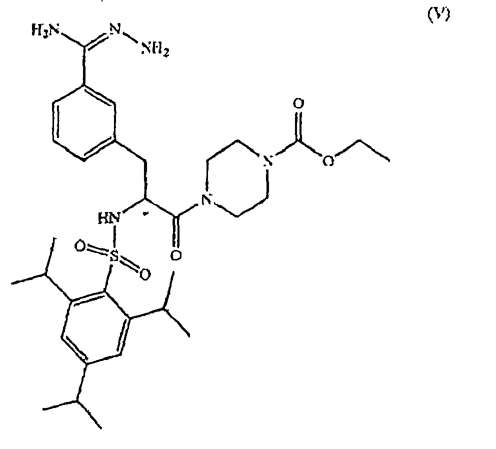 as (L)- or (D)-enantiomers, and as (E)- or (Z)-isomers or (E/Z)-mixtures, and as free bases or as the salts thereof formed with acids.

## Revendications

1. Procédé de production de dérivés de 3-hydroxyamidinophénylalanine de la formule générale (IIa) 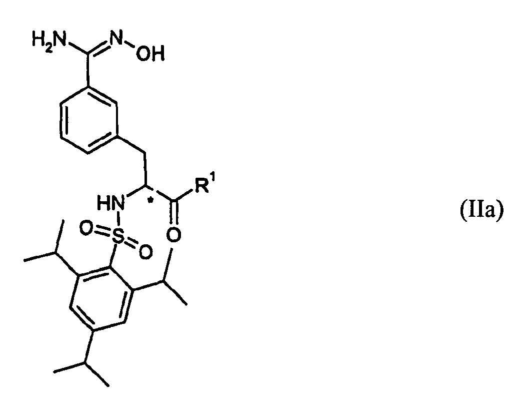 qui sont présents sous la forme d'énantiomères L ou D et sous la forme d'isomères (E) ou (Z) ou de mélanges (E/Z), et sous la forme de bases libres ou de leurs sels formés avec des acides et dans lesquels R¹ représente
(a) un groupe de la formule 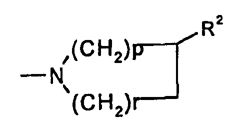 dans laquelle p = 1 et r = 2 et R² est un radical benzyloxycarbonyle, benzyl-aminocarbonyle ou 2-thiénylhydrazinocarbonyle ou bien p = 2 et r = 1 et R² est un radical éthoxycarbonyle, 2-propyloxycarbonyle, 2-propylaminocarbonyle, méthylaminocarbonyle ou méthyle ; ou
(b) un groupe de la formule 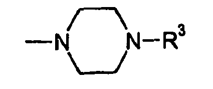
dans laquelle R³ est un radical méthoxycarbonyle, éthoxycarbonyle, benzyloxycarbonyle, diméthylaminocarbonyle, acétyle ou propionyle,
**caractérisé en ce que** l'on fait réagir un nitrile de la formule générale (III) 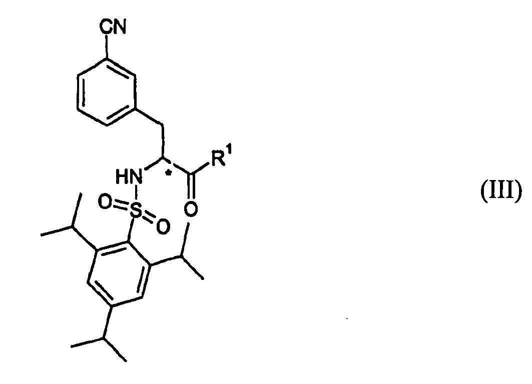 dans laquelle R¹ a la signification ci-dessus, avec du chlorhydrate d'hydroxylamine en présence de carbonate de sodium dans une solution aqueuse alcoolique à la température de reflux.

2. Procédé selon la revendication 1, dans lequel, toutefois, la réaction du nitrile de la formule générale III avec le chlorhydrate d'hydroxylamine se fait en présence de triéthylamine dans une solution alcoolique, le cas échéant en y ajoutant un autre solvant organique, à la température ambiante.

3. Procédé selon la revendication 2, **caractérisé en ce que** l'on utilise du chlorure de méthylène comme autre solvant organique.

4. Procédé selon la revendication 1, **caractérisé en ce que** l'on utilise un excès de chlorhydrate d'hydroxylamine allant jusqu'à 5 fois et **en ce que** la quantité de carbonate de sodium est de 0,5 à 0,6 équivalents molaires, ramenée au chlorhydrate d'hydroxylamine.

5. Procédé selon la revendication 1 ou 4, **caractérisé en ce que** la réaction se fait à reflux dans une solution aqueuse éthanolique.

6. Procédé de production de dérivés de 3-amidrazonophénylalanine de la formule générale (IIb) 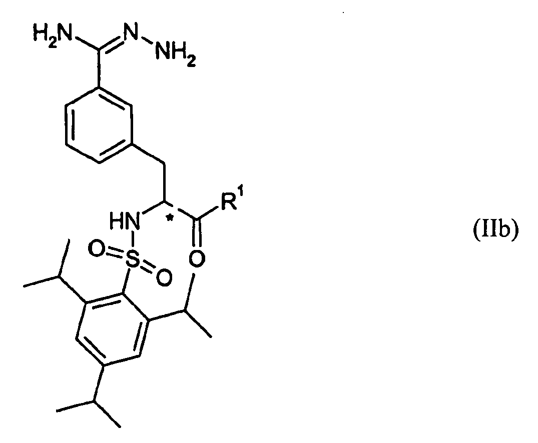 qui se présentent sous la forme d'énantiomères L ou D et sous la forme d'isomères (E) ou (Z) ou de mélanges (E/Z), et sous la forme de bases libres ou de leurs sels formés avec des acides et dans lesquels R¹ a la signification indiquée dans la revendication 1, **caractérisé en ce que** l'on chauffe à reflux un nitrile de la formule générale (III) définie dans la revendication 1 avec un excès d'hydrazine dans une solution alcoolique.

7. Procédé selon la revendication 6, **caractérisé en ce que** l'on chauffe à reflux dans une solution éthanolique pendant 2 à 20 heures, de préférence pendant 4 à 10 heures.

8. Procédé de production de dérivés de 3-amidinophénylalanine de la formule générale (I) 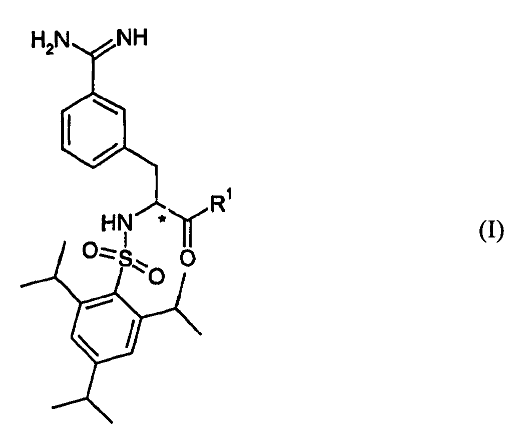 ou de leurs sels formés avec des acides, qui se présentent sous la forme d'isomères à configuration soit L, soit D, et dans lesquels R¹ a la signification indiquée dans la revendication 1, **caractérisé en ce que** l'on réduit un composé de la formule générale (IIa) définie dans la revendication 1 ou un composé de la formule générale (IIb) définie dans la revendication 6, qui se présentent sous la forme d'énantiomères L ou D et sous la forme d'isomères (E) ou (Z) ou de mélanges (E/Z) et dans lesquels R¹ a la signification indiquée dans la revendication 1, en présence de Pd/C dans une solution aqueuse alcoolique avec de l'hydrogène ou du formiate d'ammonium.

9. Procédé selon la revendication 8, dans lequel, toutefois, le composé de la formule générale (IIa) définie dans la revendication 1 ou de la formule (IIb) définie dans la revendication 6 est hydrogéné en présence de Pd/C dans une solution alcoolique additionnée d'acide acétique ou de gaz chlorhydrique sous une pression de 1 à 3 bars.

10. Variante du procédé selon la revendication 8, **caractérisée en ce que** l'on convertit les composés des formules générales (IIa) ou (IIb) avec de l'anhydride acétique en présence d'acide chlorhydrique en dérivés acétoxyamidino ou acétaminoamidino correspondants et **en ce que** l'on réduit ces derniers en présence de Pd/C dans une solution aqueuse alcoolique avec de hydrogène ou du formiate d'ammonium.

11. Procédé selon la revendication 10, dans lequel, toutefois, les dérivés acétoxyamidino resp. acétaminoamidino sont hydrogénés en présence de Pd/C dans une solution alcoolique additionnée d'acide acétique ou de gaz chlorhydrique sous une pression de 1 à 3 bars.

12. Procédé selon la revendication 10, **caractérisé en ce que** la conversion des composés des formules (IIa) ou (IIb) en dérivés acétoxyamidino ou acétaminoamidino correspondants se fait à une température de 20 à 60°C.

13. Procédé selon l'une des revendications 8, 10 et 12, **caractérisé en ce que** la réduction se fait dans une solution aqueuse éthanolique selon un ratio de 1:1 à 20:1, de préférence selon un ratio de 3:1 à 10:1, en particulier selon un ratio de 5:1.

14. Procédé selon l'une des revendications 8, 10, 12 et 13, **caractérisé en ce que** la réduction se fait en présence de 1 à 50%, de préférence en présence de 5 à 30% de Pd/C (à environ 10%).

15. Procédé selon l'une des revendications 8, 10 et 12 à 14, **caractérisé en ce que** la réduction se fait à une température de 10 à 50°C, de préférence de 20 à 30°C.

16. Procédé selon l'une des revendications 8, 10 et 12 à 15, **caractérisé en ce que** la réduction se fait avec un excès de formiate d'ammonium au moins égal à quatre fois.

17. Composés de la formule générale (IIa) 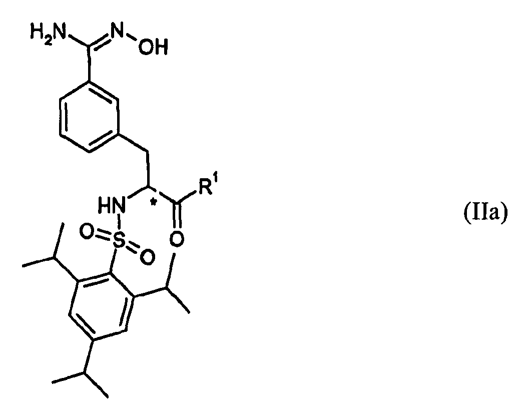 sous la forme d'énantiomères (L) ou (D) et sous la forme d'isomères (E) ou (Z) ou de mélanges (E/Z), et sous la forme de bases libres ou de leurs sels formés avec des acides, dans lesquels R¹ a la signification indiquée dans la revendication 1.

18. Composés de la formule générale (IIb) 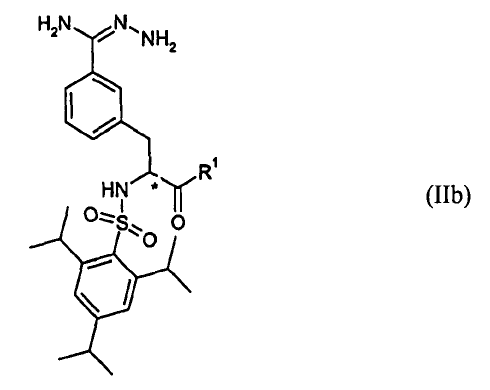 sous la forme d'énantiomères (L) ou (D) et sous la forme d'isomères (E) ou (Z) ou de mélanges (E/Z), et sous la forme de bases libres ou de leurs sels formés avec des acides, dans lesquels R¹ a la signification indiquée dans la revendication 1.

19. Composés selon la revendication 17 ou la revendication 18, des formules (IV) et (V) 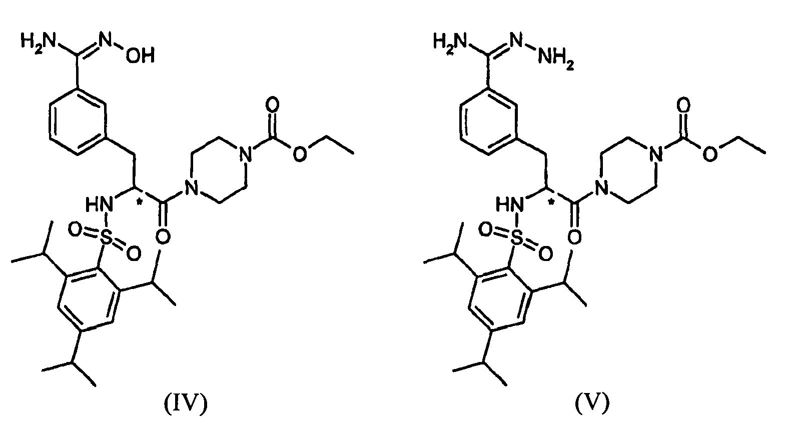 sous la forme d'énantiomères (L) ou (D) et sous la forme d'isomères (E) ou (Z) ou de mélanges (E/Z), et sous la forme de bases libres ou de leurs sels formés avec des acides.
